# EUROPEAN PATENT APPLICATION

(11) **EP 3 211 065 A1**
(43) Date of publication of application: **30.08.2017**
(21) Application number: 16000460.2
(22) Date of filing: 25.02.2016
(51) Int. Cl.: C12M 1/00, C12M 1/34

(54) **METHOD AND SYSTEM FOR CO2 DOSING FOR GROWING ALGAE**

(71) Applicant: Linde Aktiengesellschaft, 80331 München (DE)
(72) Inventor: van de Ven, Joost, 5235 DC 's-Hertogenbosch (NL); Dullstein, Stefan, 85375 Neufahrn (DE)
(74) Representative: Gellner, Bernd

(57) **Abstract**

The invention relates to a method for dosing CO₂ into a culture medium (2) for growing algae, comprising the steps of: providing a culture medium (2) containing water and algae in a container (3), and removing culture medium (2) from the container (3) and separating (7) algae from the culture medium (2). According to the invention, the culture medium (2) from which algae have been separated is fed into a reactor (4) in which CaCO₃ dissolved in the culture medium (2) is precipitated from the culture medium (2), wherein CO₂ is dosed into make-up water (5) which is then fed into the reactor (4) and mixed with the culture medium (2) therein, and wherein culture medium (2) with reduced calcium content is drawn off the reactor (4) and recycled into said container (3).

## Description

The invention is related to a method as well as to a corresponding system for dosing CO₂ into a culture medium for growing algae, wherein culture medium is removed from the container to harvest algae which are separated from the culture medium for this purpose.

It is known that CaCO₃ in an algae system can precipitate meaning loss of carbon and also settling down of algae mass. In such precipitation of CaCO₃ with algae mass, anaerobic processes can occur which again result in loss of carbon due to formation of CH₄.

Furthermore, such precipitations can settle down in places (e.g. sumps) where CO₂ is dosed and may cause a lower efficiency of solving the CO₂ in the culture medium used to grow the algae.

Therefore, based on the above, the problem underlying the present invention is to provide an improved method and system of the afore-mentioned kind.

This problem is solved by the method according to claim 1.

According thereto, the culture medium from which algae have been separated is fed into a reactor in which CaCO₃ dissolved in the culture medium is precipitated from the culture medium to separate calcium from the culture medium, wherein CO₂ is dosed into the reactor or into make-up water which is then fed into the reactor and mixed with the culture medium therein, and wherein culture medium with reduced calcium content due to the precipitation of CaCO₃ in the reactor is drawn off the reactor and then recycled into said container.

CO₂ is dosed into the culture medium since it is needed by the algae for photosynthesis of carbohydrates from CO₂ using e.g. light.

Further, the culture medium may contain nutrients like nitrogen compounds and/or phosphor compounds as well as eventually other micronutrients.

Due to the method according to the invention precipitation of CaCO₃ in the container in which the algae are grown is advantageously reduced which also reduces the precipitation of algae mass. Further, less CO₂ is bound in form of CaCO₃ in the algae producing system, i.e. in said container. Furthermore, anaerobic processes due to the settling of biomass by the calcium carbonate flocks are reduced. As a result, the efficiency of carbon capture into the (algae-) biomass increases.

The culture medium in the container contains - besides algae and water - CaCO₃ as well as CO₂, i.e.

CaCO₃ + CO₂ + H₂O <-> Ca²⁺ + 2HCO₃⁻.

The process conditions in the reactor are therefore preferably selected such that the solubility product of calcium carbonate is exceeded. As a result, calcium carbonate will be formed and precipitate (e.g. onto a seeding material, see below).

Since the recirculation culture medium from the container that was separated from the algae has a high pH (i.e. is alkaline) and contains bicarbonates and/or carbonates which according to the above equilibrium already favours precipitation of calcium carbonate, no or only a relative low dosing of an alkali, e.g. NaOH, into the reactor is sufficient to set the conditions in the reactor such that calcium carbonate precipitates in the reactor so that calcium can be removed from the recirculation culture medium that goes back to the container in which the algae are grown.

Additional dosing of NaOH, i.e.

NaOH + Ca²⁺ + 2HCO₃⁻ -> CaCO₃ + H₂O Na⁺

increases precipitation of CaCO₃.

The CO2 is either introduced directly into the reactor or into make-up water which is then fed into the reactor. In a preferred embodiment the CO2 is dosed in the make-up water of the reactor. According to the reaction

20H- + Ca2+ + CO2 --> CaC03 + H2O

CaC03 precipitation will increase with high pH and high Ca-ion content. The make up water has not a high pH and high Ca-ions content. Thus, dosing CO2 into the make-up water will avoid clogging. But direct dosing of CO2 in the reactor is also possible.

According to a preferred embodiment of the present invention, said reactor is a fluidized bed reactor, filled with a plurality of particles (i.e. seeding material), particularly sand, on which CaCO₃ is precipitated. Such a reactor is also denoted as pellet reactor.

In the fluidized bed reactor, the culture medium from the container (i.e. the feed) enters the reactor from the bottom (particularly together with an alkali to raise the pH of the water in order to increase the formation of carbonate in the water/culture medium). The saturation product of calcium carbonate will be exceeded in the reactor and calcium carbonate will come out of solution

Calcium carbonate is a crystalline solid, which will precipitate on the particles, e.g. sand, which function as seeding material/particles for the crystallization process. These particles will grow due to the ongoing crystallization of CaCO₃ and will form larger calcium carbonate pellets with time. The particles/pellets in the reactor are fluidized by the upward velocity of the culture medium from the container. This fluidization prevents the pellets from crystallizing to each other or to the walls of the reactor. Preferably, pellets in the reactor are substituted by fresh particles from time to time.

According to a further embodiment of the present invention, as already described above, an alkali, particularly NaOH, is added to the culture medium in the reactor, particularly by feeding said alkali into the reactor or into the culture medium upstream the reactor and downstream the container, in order to force CaC03 to precipitate in the reactor. According to an alternative embodiment, no such additional dosing of an alkali, particularly NaOH, is conducted. Here, the invention utilizes the fact that the recirculation culture medium from the algae container already has a high pH and high content of bicarbonates and/or carbonates.

According to a further aspect of the present invention, a system for algae production, particularly for the use in a method according to the present invention, is disclosed, wherein the system comprises: a container for receiving a culture medium comprising water and algae, a means for separating algae from the culture medium, a fluidized bed reactor for precipitating CaCO₃ from the culture medium in the fluidized bed reactor, a conduit for transporting culture medium from said means into the fluidized bed reactor, a make-up water conduit for feeding make-up water into the fluidized bed reactor, a means for dosing CO₂ into the make-up water, and a conduit for transporting culture medium from the fluidized bed reactor back into the container.

Optionally, the system further preferably comprises a means for dosing an alkali, particularly NaOH, into the culture medium in the fluidized bed reactor or into the culture medium downstream the container and upstream the fluidized bed reactor.

In the following further features and advantages of the present invention as well as preferred embodiments are described with reference to the Figures, wherein
- Fig. 1: shows a schematical illustration of a method and a system according to the invention, and
- Fig. 2: a schematical illustration of a fluidized bed reactor that can be used in the framework of the present invention.

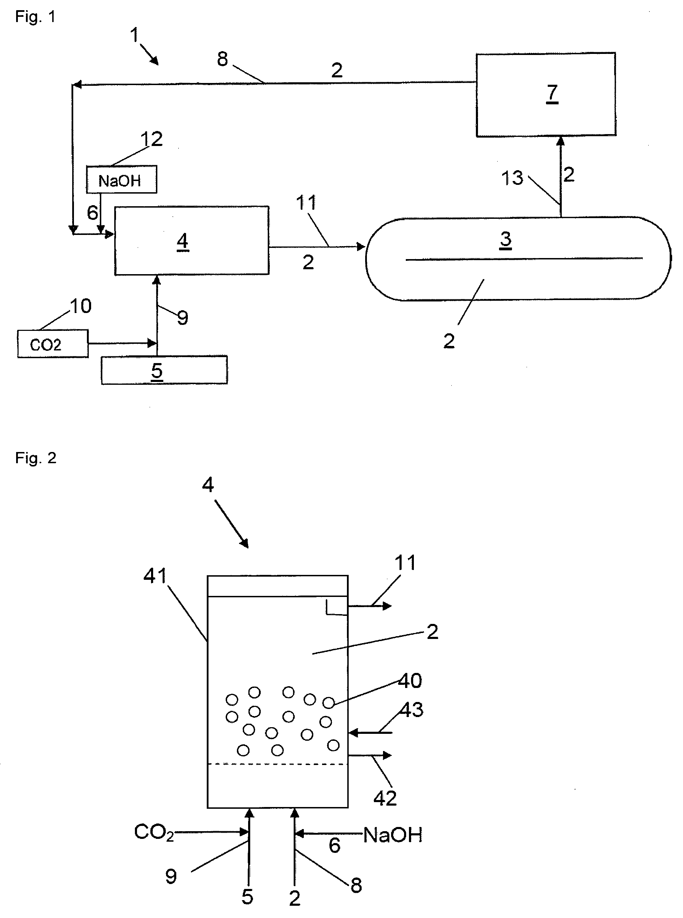

Figure 1 shows in conjunction with Fig. 2 a system 1 for growing algae as well as a corresponding method. The system 1 comprises a container 3 in which algae are grown in a culture medium 2 comprising at least water, CO₂, and also CaCO₃, wherein precipitation of the latter is to be avoided as explained above. CO₂ is dosed into the culture medium 2 for allowing the algae to produce carbohydrates from CO₂ by means of photosynthesis using e.g. light.

For harvesting algae, culture medium is removed from the container and separated from the algae in a harvesting means 7 being e.g. in flow connection with the container 3 via a conduit 13.

The culture medium 2 from which algae have been separated is then fed into a reactor 4 via a conduit 8 for recycling the culture medium 2. In order to meet the conditions for precipitation of CaC03 in the reactor 4, NaOH may be added to the culture medium 2 by a means 12 for dosing NaOH into the culture medium 2. However, since the recirculation culture medium 2 has already a high pH and content of bicarbonates and/or carbonates, NaOH dosage can be relatively low or can be even omitted.

In the reactor 4 CaCO₃ is precipitated and thus separated from the culture medium 2 which is drawn off the reactor and recycled to the container via a conduit 11 connecting the reactor 4 with the container 3. In order to provide the culture medium 2 with the necessary CO₂, the latter is dosed into make-up water 5 by a means 10 for dosing CO₂ into water, which CO₂ enriched make-up water is then fed into the reactor 4 via a conduit 9 and mixed with the culture medium 2 therein so that added CO₂ gets into the container 3 when culture medium 2 is recycled from the reactor 4 in the container 3.

As shown in Fig. 2 said reactor 4 is preferably formed as a fluidized bed reactor 4 comprising a vessel 41 that is filled with a plurality of particles 40, particularly sand, on which CaCO₃ is precipitated from the culture medium 2.

In the fluidized bed reactor 4, the culture medium 2 from the container that has been separated from the algae enters the reactor 4 preferably from the bottom via conduit 8 being in flow connection with the vessel 41 (optionally together with an alkali like NaOH to raise the pH of the water in order to increase the formation of calcium carbonate precipitate in the water/culture medium 2). Due to the conditions set in the vessel 41/reactor 4, the saturation product of calcium carbonate is exceeded and calcium carbonate comes out of solution. The calcium carbonate is a crystalline solid, which then precipitates on the particles 40, e.g. sand, of the fluidized bed in the vessel 41 which function as seeding material/particles for the crystallization process. These particles 40 will grow due to the ongoing crystallization of CaCO₃ on them and will form larger calcium carbonate pellets with time. The particles/pellets 40 in the reactor 4 are fluidized by the upward velocity of the culture medium 2 from the container. In case the pellets 40 exceed a pre-defined size at least a part of them are substituted by fresh (i.e. smaller) particles, e.g. by removing pellets 40 via conduit 42 and introducing new particles via conduit 43.

As a result; the present invention allows dosing of CO₂ in an easy manner while at the same time prevents unwanted precipitation of calcium in the container 3 where the algae are grown. Instead calcium is precipitated in form of CaCO₃ in a fluidized bed reactor 4 so that culture medium 2 can be recycled into the container having a reduced calcium content.

In the following preferred process conditions are listed.
- Temperature in the container 3 for growing the algae: 10 to 30 °C
- pH range in container 3: pH 7 to 11
- pH range of the culture medium that is recycled to the reactor 4: pH 8 - 11
- Temperature range of the culture medium in reactor 4: 10 to 30 °C
- pH range of the culture medium in reactor 4: pH 9 - 10
- Range of flow velocity of culture medium in reactor 4: 50 to 80 m/h

These conditions are advantageous but the invention is not limited to these conditions. Further, also fulfilling only some of the above conditions already improves the CO2 dosing method.

### Reference Numerals

| | |
|---|---|
| 1 | System |
| 2 | Culture medium containing at least algae and water |
| 3 | Container for growing algae |
| 4 | Reactor |
| 5 | Make-up water |
| 6 | Alkali, e.g. NaOH |
| 7 | Means for harvesting algae |
| 8, 9, 11, 13, 42, 43 | Conduit |
| 10 | Means for dosing CO₂ |
| 12 | Means for dosing an alkali, e.g. NaOH |
| 40 | Particles/pellets |
| 41 | vessel |

## Claims

1. Method for dosing CO₂ into a culture medium (2) for growing algae, comprising the steps of:
- providing a culture medium (2) containing water and algae in a container (3), and
- removing culture medium (2) from the container (3) and separating (7) algae from the culture medium (2),
**characterized in that**
the culture medium (2) from which algae have been separated is fed into a reactor (4) in which CaCO₃ dissolved in the culture medium (2) is precipitated from the culture medium (2), wherein CO₂ is dosed into the reactor or into make-up water (5) which is then fed into the reactor (4) and mixed with the culture medium (2) therein, and wherein culture medium (2) with reduced calcium content is drawn off the reactor (4) and recycled into said container (3).

2. Method according to claim 1, **characterized in that** said reactor (4) is a fluidized bed reactor, filled with a plurality of particles (40), particularly sand, on which CaCO₃ is precipitated.

3. Method according to claim 1 or 2, **characterized in that** an alkali (6), particularly NaOH, is added to the culture medium (2) in the reactor (4), particularly by feeding said alkali (6) into the reactor (4) or into the culture medium (2) upstream the reactor (4) and downstream the container (3).

4. Method according to claim 1 or 2, **characterized in that** no additional alkali, particularly NaOH, is added to the recycled culture medium.

5. System for algae production, particularly for the use in a method according to one of the preceding claims, comprising:
- a container (3) for receiving a culture medium (2) comprising water and algae, and
- a means (7) for separating algae from the culture medium (2),
**characterized by**
- a fluidized bed reactor (4) for precipitating CaCO₃ from the culture medium (2) in the fluidized bed reactor (4),
- a conduit (8) for transporting culture medium (2) from said means (7) into the fluidized bed reactor (4),
- a make-up water conduit (9) for feeding make-up water (5) into the fluidized bed reactor (4),
- a means (10) for dosing CO₂ into the make-up water (5), and
- a conduit (11) for transporting culture medium (2) from the fluidized bed reactor (4) back into the container (2).

6. System according to claim 5, **characterized in that** the system (1) comprises a means (12) for dosing an alkali, particularly NaOH, into the culture medium (2) in the fluidized bed reactor (4) or into the culture medium (2) downstream the container (3) and upstream the fluidized bed reactor (4).
